# EUROPÄISCHE PATENTSCHRIFT

(11) **EP 2 023 911 B1**
(45) Veröffentlichungstag und Bekanntmachung des Hinweises auf die Patenterteilung: **03.07.2013**
(21) Anmeldenummer: 07722325.3
(22) Anmeldetag: 27.04.2007
(51) Int. Cl.: A61K 31/133, A61K 31/164, A61K 31/19, A61K 31/4164, A61K 31/6615, A61P 1/16, A61P 1/18, A61P 3/10, A61P 25/32, A61P 31/14, A61P 35/00, A61P 39/02, A61P 43/00

(54) **VERBINDUNGEN A-R-X ZUR HERSTELLUNG VON PHARMAZEUTISCHEN ZUBEREITUNGEN**
COMPOUNDS A-R-X FOR THE MANUFACTURE OF PHARMACEUTICAL PREPARATIONS
UTILISATION DE COMPOSÉS DE FORMULE A-R-X OU DE SELS PHARMACEUTIQUEMENT ACCEPTABLES DESDITS COMPOSÉS DANS LA PRODUCTION D'UNE PRÉPARATION PHARMACEUTIQUE

(30) Priorität: 28.04.2006 DE 102006019906
(43) Veröffentlichungstag der Anmeldung: 18.02.2009
(73) Patentinhaber: Universität Ulm, 89081 Ulm (DE); Haehner, Thomas, 89160 Dornstadt (DE)
(72) Erfinder: Mueller-Enoch, Dieter, 89160 Dornstadt (DE); Haehner, Thomas, 89160 Dornstadt (DE)
(74) Vertreter: Dinné, Erlend
(86) Internationale Anmeldenummer: PCT/DE2007/000768
(87) Internationale Veröffentlichungsnummer: WO 2007/124734

(56) Entgegenhaltungen:
- EP-A1- 0 567 653
- WO-A-2006/045298
- DE-A1- 3 323 264
- GB-A- 2 016 452
- PARANG KEYKAVOUS ET AL: "IN VITRO ANTIVIRAL ACTIVITIES OF MYRISTIC ACID ANALOGS AGAINST HUMAN IMMUNODEFICIENCY AND HEPATITIS B VIRUSES" ANTIVIRAL RESEARCH, ELSEVIER SCIENCE BV., AMSTERDAM, NL, Bd. 34, Nr. 3, 1997, Seiten 75-90, XP008072204 ISSN: 0166-3542
- HAEHNER, T ET AL.: "Inhibition of Cytochrome P450 Mediated Enzyme Activity by Alkylphosphocholines" Z. NATURFORSCH., Bd. 59, Nr. 7-8, 2004, Seiten 599-605, XP009090615

## Beschreibung

Die Erfindung betrifft 12-imidazolyl-1-dodecanol und seine pharmazeutisch akzeptablen Salze sowie seine pharmazeutische Zubereitungen zur Vorbeugung oder Behandlung von Erkrankungen bzw. unerwünschten Zuständen, insbesonders am Menschen .

Übermäßiger dauerhafter Alkoholkonsum führt häufig zu einer Lebererkrankung, der so genannten Fettleber, die sich zu einer Leberentzündung bzw. Hepatitis und im Spätstadium zu einer Leberzirrhose weiter entwickeln kann. Dabei hängt das Risiko und das Ausmaß des jeweiligen Leberschadens direkt von der Menge und der Dauer des Alkoholkonsums ab, wobei das Risiko individuell unterschiedlich ist Eine alkoholbedingte Entzündung der Leber (Alkoholhepatitis) stellt eine ggf. lebensbedrohliche Erkrankung dar, die mit Fleber, Gelbsucht sowie einer Erhöhung der weißen Blutkörperchen einhergehen kann. Derartige alkoholbedingte Leberentzündungen sind durch völlige Alkoholabstinenz ausheilbar, Vernarbungen bei der Leberzirrhose ausgenommen.

Neben dieser alkoholinduzierten sogenannten Fettieberhepatitis oder alkohollschen Steato-Hepatitis (ASH) entstehen auch Hepatiden bei Personen, die keinen Alkoholmissbrauch betreiben, oder überhaupt keinen Alkohol zu sich nehmen. Solche Hepatiden werden beispielsweise durch Umweltgifte, beispielsweise bei Arbeiten in Lackierwerken und/oder auch durch Medikamente induziert.

Es Ist bekannt, dass Oxidationsprozesse im Stoffwechsel mit Hilfe von Cytochromen erfolgen. Bei den Cytochromen handelt es sich um eine Vielzahl verschiedener Enzyme, deren aktives Zentrum eine Hämstruktur aufweist. Sie katalysieren bei einer Vielzahl von Oxidations- und Hydroxylierungsreaktionen den Transfer von Elektronen auf einen Akzeptor.

Eine wichtige Rolle spielen z.B. die Cytochrome der P450-Familie (CYP-450). Hierbei handelt es sich um Monooxygenasen, die ubiquitär sind und zu den wichtigsten Enzymen des Metabolismus hydrophober körperfremder Stoffe und der Modifikation hydrophober Hormone, der Steroide, gehören.

Eine der Hauptaufgaben der Cytochrom-P450-Enzyme ist es, körperfremde Stoffe durch Hydroxyilerung zu solubilisieren und auf diese Weise der renalen Exkretion zuzuführen. Cytochrom-P450-Enzyme spielen daher eine wichtige Rolle bei der Entgiftung.

Es wird geschätzt, dass ca. die Hälfte aller derzeitigen Arzneistoffe durch Cytochrom-P450-Enzyme der Leber hydroxyliert wird. Die Verweildauer vieler Arzneistoffe im Körper wird daher durch die Aktivität von Cytochrom-P450-Enzymen z. T. erheblich vermindert. Die überwiegende Menge an Cytochrom P450 findet sich bei Säugetieren in der Leber, da diese das zentrate Entgiftungsorgan darstellt. Cytochrom P450 liegt in der Regel in an die Membran des endoplasmatischen Reticulums gebundener Form vor.

Auch bei der Vermittlung der Resistenz von Insekten gegen Insektizide und der Pflanzen gegen Herbizide spielen Cytochrom-P450-Enzyme eine Schlüsselrolle.

In seiner Grundstruktur weist Cytochrom P450 eine sechsfach koordinierte Hämgruppe auf, an der eine Reaktion folgenden Schemas

RH +O₂+2H⁺ + 2 e⁻→ ROH + H₂O

katalysiert wird. Dabei werden die beiden für diese Reaktion erforderlichen Elektronen z. B. von NADPH-Cytochrom P450-Reduktase bereitgestelit, die mit dem Enzymkomplex assoziiert sind. Auf diese Weise entstehen am P450 unter anderem auch cytotoxische, reaktive Sauerstoffspezies (ROS).

Es ist bekannt, dass sowohl bei Alkoholkonsum als auch bei der nichtalkohollschen Fettleberhepatitis als auch bei Pankreatitis die Synthese des Cytochroms P450-2E1 induziert wird. Die Funktion und Wirkungsweise dieser von anderen Cytochromen stark verschiedenen isoform ist beispielsweise von M. H. Wang et al. In Archives of Biochemistry and Biophysics, (1995) Vol. 317, Seite 299 bis 304 beschrieben. Danach weist das Enzym einen ca. 15 Å langen Kanal auf, an dessen Ende das reaktive Zentrum mit einem ein zentrales Elsenatom aufweisenden Hämring sitzt.

Seit längerem wurde vermutet, dass auch Chemotherapeutika, wie sie In der Krebstherapie zum Einsatz kommen, durch Cytochrom P450-Enzyme abgebaut werden.

In einer jüngeren Studie von Jiang et al. ("Cytochrome P450 2J2 Promotes the Neoplastic Phenotype of Carcinoma Cells and is Up-regulated in Human Tumors" In Cancer Res. 2005, 65: 4707-4715) wurde jedoch erstmals gezelgt, dass Cytochrom P450 sogar eine krebsfördemde Wirkung haben kann.

Es wurde gezeigt, dass die Genexprassion von Cytochrom P450 2J2 In menschlichen Tumoren hochreguliert wird. Bei Cytochrom P450 2J2 handelt es sich um eine Epoxygenase, die das Substrat Arachidonsäure in vier verschiedene isomere Epoxyeicosatrienolde Säuren (EET) überführt. In der Studie wurde ferner gezeigt, dass EET eine apoptosehemmende Wirkung aufweisen, da sie die Tumorzelien vor der Wirkung von Tumomekrosefaktoren schützen, und auf diese Weise die Lebensdauer der Krebszelien erhöhen. Überdies fördern sie die Mitose sowie die Proliferation von Tumorzellen.

Es konnte ebenfalls gezeigt werden, dass EETs die Angiogenese, d. h. die Bildung neuer Blutgefäße fördern. Dieser Vorgang spielt eine wichtige Rolle beim Wachstum von Tumoren (Pozzl A. et al. "Characterization of 5,6- and 8,9-Epoxyeicosatrienoic Acids (5,6- and 8,9-EET) as Potent In "Vivo Angiogenic Lipids", J. Biol. Chem. 280:27138-27146. 2005).

In einer Schrift von Schattenberg et al. ("Hepatocyte CYP2E1 overexpression and steatohepatitis lead to impaired hepatic Insulin signalling" in J. Biol. Chem. 2005; 280: 9887-9894) wird hingegen erstmals eine Überexpression von Cytochrom P450 mit Diabetes In Verbindung gebracht.

Müller-Enoch et al. beschrelben In Z. Naturforsch. (2001) 56c, Seite 1082-1090 die Inhibierung von Cytochrom-P450 2B1 der Ratte mittels Lysophosphatidylcholinen, Lysophosphatidylinositol sowie Arachidon- und Oleinsäuren bzw. mittels Monoacylglycerolen, Monooleylglycerolen, und Monopalmitoylglycerolen.

Des weiteren wird von T. Haehner, D. Müller-Enoch et al. in Z. Naturforschung (2004) 59c, Seite 599-605 der Einfluss von einkettigen Lipidmolekülen auf die Aktivität der isoform Cytochrom P 450 2B1 der Ratte beschrieben.

Aufgabe der vorliegenden Erfindung ist es, Mittel zur Herstellung einer pharmazeutischen Zubereitung bereit zu stellen, die sich zur Vorbeugung oder Behandlung von Krebserkrankungen, pathologischer Folgen des Alkoholmissbrauch, viraler Hepatitis, Steato-Hepatltis, akuter und chronischer Pankreatitis, toxischer Niarenerkrankungen, hepatischer Insulinresistenz bei Diabetes mellitus, Leberschäden bei Morbus Wilson und Siderosen, ischämie-Reparfusionsschäden, zur Verwendung als Antidot gegen Umweltgifte und Medikamenten-intoxikation, zur Verlängerung der Verweildauer von Medikamenten im Organismus, oder zur Bekämpfung toxischer Nebenwirkungen bei der Verabreichung von Chemotherapeutika eignet.

Diese Aufgabe wird mit einer Verbindung gemäß der in den Ansprüchen defi-. nierten Merkmale erfüllt

Es wurde nämlich überraschenderweise gefunden, dass sich die vorgenannten Erkrankungen mit derartigen Verbindungen therapieren lassen. Diese Verbindungen hemmen die Blidung von reaktiven Sauerstoffspezies (ROS), insbesonders Sauerstoffradikalen wie das Superoxidanion (O₂^{•-}) sowie Wasserstoffperoxid (H₂O₂), die nicht In einer direkten Redoxreaktion verbraucht werden am Cytochrom P450, insbesonders an den isoformen der Genfamilie 2, speziell 2E1, sowie 2J2.

Ebenso wurde festgestellt, dass diese Verbindung auch die Umwandlung von Arachidonsäure In Isomere Epoxyeicosatrienoide Säuren hemmt. Ferner wurde gezeigt, dass auch die Hydroxylierung körperfremder Stoffe bei Gabe einer solchen Verbindung gehemmt werden kann.

Die erflndungsgemäße Verbindung ist 12- imidazolyl-1-dodecanol.

Alternativ können auch seine pharmazeutisch akzeptable Salze verwendet werden.

Bei der erfindungsgemäßen Verwendung zeigen die Verbindungen eine Wirkung bei der Krebstherapie, wobei unter anderem die Umwandlung von Arachldonsäure in epoxyeicosatrienoide Säuren gehemmt wird. Letztere fördern die Zellteilung und -Proliferation und hemmen die Apoptose von Tumorzellen. Ebenso wird bei Applikation einer solchen Verbindung die Hydroxylierung von Chemotherapeutika, die letztlich zu deren Exkretion und damit zu deren Inaktivierung führt, gehemmt wird. Eine solche Verbindung kann damit sowohl für eine direkte als auch für eine adjuvante Tumortherapie verwendet werden. Aus diesem Grunde ist die oben genannte bevorzugte Ausführungsform, die einen gezielten Transport In das pathogene Gewebe ermöglicht, besonders erfolgversprechend.

Erfindungsgemäß ist weiterhin eine pharmazeutische Zubereitung vorgesehen, enthaltend eine erfindungsgemäße Verbindung In einem pharmazeutisch akzeptablen Träger.

Mögliche Indikationen für eine erfindungsgemäße Verbindung bzw. deren pharmazeutische Zubereitung liegen überdies in der Behandlung der Folgen von Alkoholmissbrauch. Dies sind insbesonders Leberschäden oder auch andere alkoholbedingte, entzündliche Prozesse. Neben den rein alkoholisch bedingten Leberschäden verursachen auch ernährungsbedingte und endokrine Faktoren, wie z. B. Adipositas, aber auch Diabetes mellitus und Hyperlipidämie ebenfalls alkoholunabhängig eine schwere Leberseädigung, die über eine Fettieberhepatitis (nicht-alkoholische Steato-Hepatitis = NASH) bis hin zur Leberzirrhose reichen kann. Derartige alkoholische und nicht-alkoholische Fettiebererkrankungen gehen häufig mit einer viralen Infektion der Leber einher. Dabei kann es zu einem sehr schnellen Fortschreiten der Erkrankung kommen. Es hat sich gezeigt, dass alle vorgenannten Erkrankungen bzw. deren Ursachen oder Folgen mit den erfindungsgemäßen Verbindungen behandelbar sind.

Es wurde auch gefunden, dass diese Substanzen sich gut zur Behandlung von Entzündungen der Bauchspeicheldrüse eignen. Derartige Entzündungen bzw. Pankreatitiden können neben Alkoholmissbrauch auch durch toxische Substanzen hervorgerufen werden. Hierzu zählen insbesonders Umweltgifte, wie Berufschemikalien oder auch Medikamente. Auch virale Infektionen oder metabolisch-endokrine Faktoren können derartige Pankreasentzündungen verursachen, wobei in allen Fällen reaktive Sauerstoffspezies an der Krankheltsentstehung und am Fortschreiten der Erkrankung beteiligt sind.

Auch bei der Behandlung von Diabetes mellitus, sowohl bei Diabetes mellitus Typ 2 als auch Typ 1, hat sich das erfindungsgemäße Pharmazeutikum als geeignet erwiesen.

Auch toxische Nierenerkrankungen, sowie andere Erkrankungen, wie sie z. B. durch Nebenwirkungen bei der Verabreichung von Chemotherapeutika, insbesondere Zellgiften, wie Metall-Komplexe wie Cisplatin, Carboplatin, Titanocendichlorid oder Goldkomplexe hervorgerufen werden, sind mit dem erfindungsgemäßen Arznelmittel zu behandeln. Hierbei hat es sich insbesondere gezeigt, dass sich die Organotoxizität von Metall-Komplexen oder auch anderen toxischen Mitteln, wie halogenlerten Kohlenwasserstoffen und zwar sowohl monowie auch polyhalogenierten Kohlenwasserstoffen darunter auch Dampfnarkotika vom Halothan-Typ, sowie entsprechende aromatische Kohlenwasserstoffe, Nitrosamine, Acrylamid oder Arzneimittel, wie Paracetamol, Methotrexat, Isoniazid oder Aminoglykorid-Antiblotika oder Rönigen-Kontrastmittel verhindern lässt. Das erfindungsgemäße Arzneimittel ist somit auch zur Behandlung der Organotoxizität von Umweltgiften, insbesonders als Antidot hierfür an Organen, wie Leber, Niere, zentrales Nervensystem, Pankreas etc. geeignet.

Es ennöglicht so z.B. auch eine höhere Dosierung von Cytostatika in der Krebstherapie, und kann auch vor diesem Hintergrund als adjuvante Therapie die Erfolgssaussichten der Chemotherapie erhöhen.

Ganz besonders hat es sich als geeignet erwiesen, Schäden zu vermeiden, die durch Reperfusion von biologischen Geweben entstehen, wie beispielsweise nach Organinfarkten, insbesonders dem Herzen, sowie dem Hirn (Herzinfarkt, Schlaganfall). So konnte beispielsweise in Tierexperimenten gezeigt werden, dass derartige Reperfusionsschäden zu 60 - 80 % der Gewebszerstörung beitragen bzw. dass die Ausbreitung des Gewebstodes um diesen Faktor verringert werden kann. Seit längerem weiß man, dass eine Hauptursache der Reperfusionsschäden Sauerstoffradikale sind, die während der Ischämie gebildet werden.

Das erfindungsgemäße Mittel ist somit auch besonders geelgnet zur Vermeidung von Reperfusionsachäden bei transplantierten Organen. Derartige Organe werden bis zu ihrer Transplantation In den Körper eines neuen Empfängers in einer gekühlten Nährlösung gehalten. Nach der Transplantation werden diese dann nach Anschluss an das Kreistaufsystem des Empfängers wieder mit Körperflüssigkeiten durchströmt, was zu Reperfusionsschäden führt. Durch eine Verabreichung des erfindungsgemäßen Mittels vor und während der Lagerung, sowie kurz vor dem implantieren In den Empfängerorganismus, kann auch dieses wichtige Transplantationsproblem gelöst werden.

Eine Akkumulation des Übergangsmetalls Elsen (bzw. von Kupfer bei M. Wilson) verursacht eine Potenzierung der durch Cytochrom P450 verursachten oxidativen Schäden. Neben einer Elsenüberladung im Rahmen von Slderosen ist bekannt, dass sich bei ischämie-Reperfusions-Schäden ein deutlicher Anstieg der Intrazellulären Konzentration an freiem Eisen auftritt. Ein intrazellulärer Eisenanstieg wird auch durch toxische Verbindungen, wie z. B. Cisplatin oder halogenierte Kohlenwasserstoffe oder durch virale Hepatitiden hervorgerufen. Betroffen ist vor allem die Leber, aber auch z. B. bei Cisplatin, das renal eliminiert wird, die Niere. Blochemische Grundlage der oxidativen Schäden durch das Übergangsmetall Elsen (bzw. bei M. Wilson Kupfer) stellt die Fenton-Reaktion dar.

H₂O₂ + Fe²⁺ → Fe³⁺ + HO⁻ + HO⁻

Das im Rahmen des Cytochrom P450-Reaktionszyklus entstehende H₂O₂ (Redoxpotential +0,32 Volt) wird hierbei In das äußerst stark oxidierende Hydroxylradikal HO* (Redoxpotential +2,31 Volt) überführt.

Eine Erhöhung der Konzentration an frelem Elsen im Rahmen der durch Cisplatin bedingten Nephropathie wird z. B. in einer Schrift von Baliga et al. ("Role of cytochrome P450 as a source of catalytic iron In cisptatin-induced nephrotoxicity", Kidney Int. 1998; 54: 1562-1569) beschrieben. Eine Eisenerhöhung bei Ischämie-Reperfusionsschäden oder bei viraler Hepatitis wird z. B. durch Paller et al. ("Cytochrome P450 mediates tissue damaging hydroxyl radical formation during reoxygenation of the kidney", Proc. Natl. Acad. Sci. USA 1994; 91:7002-7006) bzw. durch Chapoutot et al. (Liver iron excess In patients with hepatotocellular carcinoma developed on viral C cirrhosis", Gut 2000; 46:711-714).

Die erfindungsgemäßen Substanzen haben sich insbesonders als inhibitoren der humanen isoformen der Genfamilie 2 des Cytochroms P450 und zwar insbesonders der isoformen 2E1 und 2J2 und durch diese verursachten Erkrankungen erwiesen.

In einer besonders zweckmäßigen Ausgestaltung der Erfindung ist vorgesehen, dass die pharmazeutische Zubereitung In Uposomen eingebunden ist. Aufgrund der Tatsache, dass die der Zubereitung zugrunde liegenden Verbindungen lange aliphatische Reste aufweisen, ist deren Einbindung in Uposomen eine sehr geeignete Verabreichungsform. Solche Liposomen eignen sich für eine Intravenöse, intramuskuläre, intraperitoneale, percutane oder auch orale Verabreichung. Ebenso ist auch eine Verabreichung als Aerosol geeignet.

Die erfindungsgemäßen Verbindungen können jedoch auch als solche direkt verabreicht werden. Auch In diesem Fall sind die oben genannten Verabreichungsarten geeignet.

12-Imidazolyl-1-dodecanol und 1-Imidazolyidodecan wird gemäß einem Verfahren synthetisiert, das in der Schrift von Lu et al. ("Heme-coordinating analogs of lauric acid as inhibibors of fatty acid ω-hydroxylation", Archives of Biochemistry and Biophysics 1997, 337:1-7) beschrieben ist.

Hierzu werden 12-Bromo-1-dodecanol und Imidazol im Molverhältnis 1:3 bei 80° C fünf Stunden lang erhitzt. Das Rohprodukt wird zwischen Wasser und Dichlormethan verteilt. Die organische Phase wird über Na₂SO₄ getrocknet und eingedampft. Das 12-Imidazolyl-1-dodecanol wird aus Benzol/n-Hexan umkristallisiert

### Toxizität

Die akute Toxizität von 12-Imidazolyl-1-dodecanol (Substanz 1) wurde an männlichen CD Ratten getestet. Dabei ergab sich für das 12-Imidazolyl-1-dodecanol ein LD50 (14 Tage) von 1000 mg/kg b.w., p.o.

| | |
|---|---|
| Erste Intoleranzreaktion: | Substanz 1: 1000 mg/kg b.w., p.o. |
| Kein Effekt bei Dosisgröße: | Substanz 1: 500 mg/kg b.w., p.o. |

### Bestimmung der antineoplastischen Wirkung von 12-imidazolyl-1-dodecanol

Hierzu wurden vier Krebszell-Linien In jeweils "24-Well plates" eingesät und 24 Stunden wachsen gelassen. Anschlleßend gab man zu den Zellsuspensionen verschiedene Konzentrationen der Testsubstanz 12-Imidazolyl-1-dodecanol, gelöst In DMSO, hinzu. Die DMSO-Konzentration im Zelimedium betrug Jeweils 0,1%. Diese DMSO-Konzentration erwies sich im Kontrollversuch als nicht toxisch, die Zellzählungen wurde jeweils nach viertägiger Inkubationszeit durchgeführt. In allen Fallen wurde ein starke Inhibition der Krebszellen-Proliferation festgestellt.

Folgende halbmaximale Inhibitoriconzentrationen, IC50-Werte von 12-Imidazolyl-1-dodecanol wurden ermittelt:

| | |
|---|---|
| HepG2 (Leberzellen) | 50 nM |
| Panc-1 (Pankreaszellen) | 50 nM |
| PC-3 (Prostatamllen) | 50 nM |
| SW620 (Dickdarmzellen) | 100 nM |

In allen Krebszeil-Linien wurde ein starker Andneoplastischer Effekt des Inhibitors festgestellt.

### Evaluation der Cytotoxizität von 12-Imidazolyl-1-dodecanol

Die halbmaximale Cytotoxizität von 12-Imidazolyl-1-dodecanol wurde mit dem LDH-Cytotoxizitäts-Test bestimmt.
Dabei wurden folgende Werte gemessen:

| | |
|---|---|
| MRC-5 (Lungen-Fibroblastzellen) | = 500 µM |
| Panc-1 (Pankreaszellen) | = 500 µM |

Ein Vergleich der halbmaximalen Cytotoxizität von 500 µM, mit der halbmaximalen Inhibitorkonstanten IC50=50 µM für Panc-1, engibt einen Differenzfaktor von 10.000. Daraus lässt sich schlleßen, dass 12-Imidazolyl-1-dodecanol ein hochwirksamer Inhibitor der Krebszellen-Proliferation mit relativ geringer Cytotoxizität ist.
Mit anderen Worten, die erfindungsgemäßen Substanzen besitzen ein breites therapeutisches Fenster.

### Hemmung der Bewegungsaktivität von HepG2-Zellen durch 12-Imidazolyl-1-dodecanol

Migrationstestbedingungen: HepG2-Leberkrebszellen wurden in eine KollagenMatrix eingebettet. 30 Einzeizellen wurden kontinulerlich über 900 min. fotographisch kontrolliert. Der durchschnittliche Prozentsatz der wandernde Zellen wurde ermittelt.

Ergebnis 1: Nicht behandelte Kontroll-HepG2-Zellen weisen eine mittlere Migrationsaktivität von 15% auf. Bei Zusatz von 1 µM bzw. 10 µM 12-Imidazolyl-1-dodecanol zum Matrixmedium sank die Migrationsaktivität der Zellen um 50 % bzw. 75 % ab.

Ergebnis 2: Da HepG2-Zellen eine starke Expression von Insulinrezeptoren aufweisen, wurde der Einfluss von Insulin auf die Migrationsaktivität mit und ohne 1 2-Imidazolyl-1-dodecanol bestimmt.

| **HepG2-Zellen** | **Mittlere Migrationsaktiviät** |
|---|---|
| Kontrolle | 15 % |
| Zusatz von insulin (100 ng/ml) | 38 % |
| Zusatz von Insulin (100 ng/ml) und 12-Imidazolyl-1-dodecanol (10 µM) | 15 % |

Insulin bewirkt einen 2,5-fachen Anstieg der Migrationsaktivität. Die Wirksubstanz blockiert diesen Anstieg vollständig nach 450 min. Beobachtungszeit (erste Hälfte der Beobachtungszeit). Nach 900 min. (zweite Hälfte der Beobachtungszeit) ist der Anstieg negativ. Die mittiere Migrationsaktivität liegt dann 13% unter der der Kontrolle.

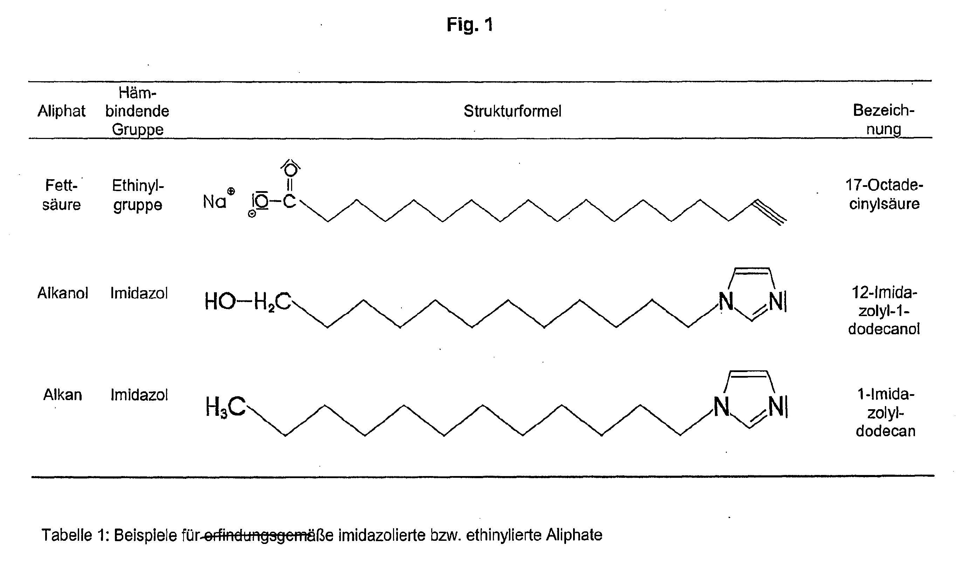

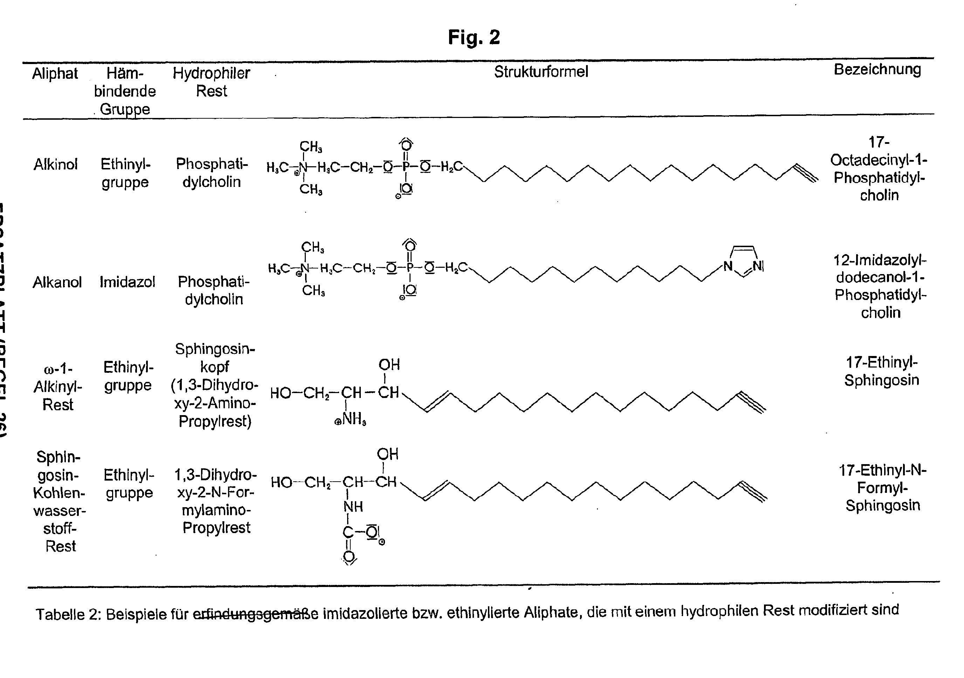

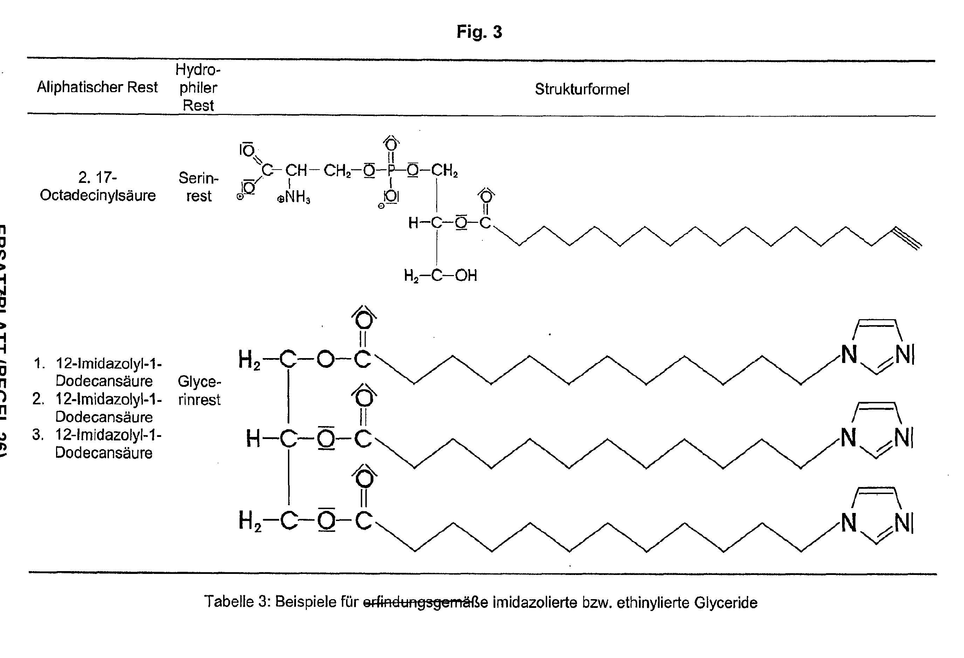

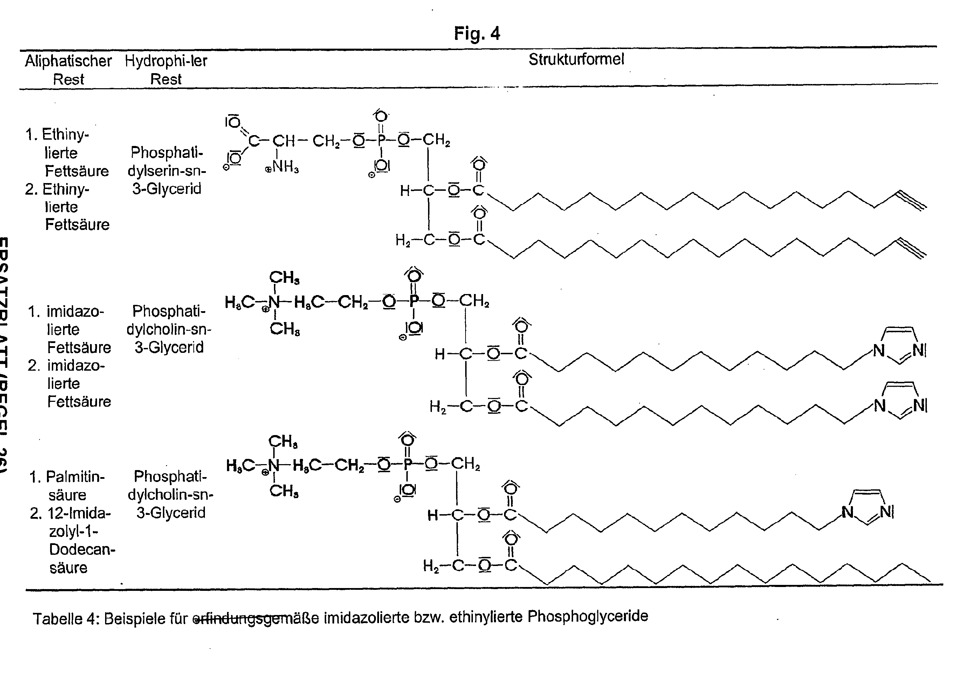

## Patentansprüche

1. Verwendung von 12-Imidazolyl-1-dodecanol oder einem pharmazeutisch akzeptablen Salz davon zur Herstellung einer pharmazeutischen Zubereitung.

2. Verwendung nach Anspruch 1 zur Herstellung einer pharmazeutischen Zubereitung zur Vorbeugung oder Behandlung von Krebserkrankungen, pathologischen Folgen des Alkoholmissbrauchs, viraler Hepatitis, Steato-Hepatitis, akuter und chronischer Pankreatitis, toxischer Nierenerkrankungen, hepatischer Insulinresistenz bei Diabetes mellitus, Leberschäden bei Morbus Wilson und Siderosen, Ischämie-Reperfusionsschäden, als Antidote gegen Umweltgifte und Medikamentenintoxikation, zur Verlängerung der Verweildauer von Medikamenten im Organismus, oder zur Bekämpfung toxischer Nebenwirkungen bei der Verabreichung von Chemotherapeutica.

3. Verwendung nach Anspruch 1 zur Herstellung einer pharmazeutischen Zubereitung zur Behandlung von Hyperlipidämie.

4. Verwendung nach Anspruch 1 zur Herstellung einer pharmazeutischen Zubereitung zur Vermeidung von Reperfusionsschäden bei transplantierten Organen, insbesondere vor und während der Lagerung, sowie kurz vor dem Implantieren in den Empfängerorganismus.

5. Pharmazeutische Zubereitung enthaltend 12-Imidazolyl-1-dodecanol oder ein pharmazeutisch akzeptables Salz davon in einem pharmazeutisch akzeptablen Träger.

6. Pharmazeutische Zubereitung gemäß Anspruch 5, **dadurch gekennzeichnet, dass** die Zubereitung in Liposomen eingebunden ist.

7. 12-Imidazolyl-1-dodecanol oder ein pharmazeutisch akzeptables Salz davon.

## Claims

1. Use of 12-imidazolyl-1-dodecanol or a pharmaceutically acceptable salt thereof for preparing a pharmaceutical preparation.

2. Use according to Claim 1 for preparing a pharmaceutical preparation for the prevention or treatment of cancer, pathological sequelae of alcohol abuse, viral hepatitis, steatohepatitis, acute and chronic pancreatitis, toxic kidney disorders, hepatic insulin resistance in diabetes mellitus, liver damage in Wilson's Disease and siderosis, ischemia reperfusion damage, as an antidote for environmental toxins and medicament intoxication, for prolonging the residence time of medicaments in the organism or for controlling toxic side effects when administering chemotherapeutics.

3. Use according to Claim 1 for preparing a pharmaceutical preparation for treating hyperlipidaemia.

4. Use according to Claim 1 for preparing a pharmaceutical preparation for preventing reperfusion damage in transplanted organs, in particular prior to and during storage, and also immediately before implantation into the recipient organism.

5. Pharmaceutical preparation, comprising 12-imidazolyl-1-dodecanol or a pharmaceutically acceptable salt thereof in a pharmaceutically acceptable carrier.

6. Pharmaceutical preparation according to Claim 5, **characterized in that** the preparation is incorporated into liposomes.

7. 12-Imidazolyl-1-dodecanol or a pharmaceutically acceptable salt thereof.

## Revendications

1. Utilisation du 12-imidazolyl-1-dodécanol ou d'un sel pharmaceutiquement acceptable de celui-ci pour la fabrication d'une préparation pharmaceutique.

2. Utilisation selon la revendication 1 pour la fabrication d'une préparation pharmaceutique destinée à la prévention ou au traitement de maladies cancéreuses, de suites pathologiques de l'abus d'alcool, de l'hépatite virale, de la stéato-hépatite, de la pancréatite aigüe et chronique, de néphropathies toxiques, de la résistance hépatique à l'insuline dans le diabète sucré, de lésions hépatiques dans la maladie de Wilson et les sidéroses, de lésions d'ischémie-reperfusion, comme antidote à des poisons environnementaux et à une intoxication médicamenteuse, pour prolonger le temps de séjour de médicaments dans l'organisme, ou pour lutter contre des effets secondaires toxiques dans l'administration d'agents chimiothérapeutiques.

3. Utilisation selon la revendication 1 pour la fabrication d'une préparation pharmaceutique destinée au traitement de l'hyperlipidémie.

4. Utilisation selon la revendication 1 pour la fabrication d'une préparation pharmaceutique destinée à réduire les lésions de reperfusion dans des organes transplantés, en particulier avant et pendant la conservation, ainsi que peu de temps avant l'implantation dans l'organisme receveur.

5. Préparation pharmaceutique contenant du 12-imidazolyl-1-dodécanol ou un sel pharmaceutiquement acceptable de celui-ci dans un véhicule pharmaceutiquement acceptable.

6. Préparation pharmaceutique selon la revendication 5, **caractérisée en ce que** la préparation est incluse dans des liposomes.

7. 12-imidazolyl-1-dodécanol ou un sel pharmaceutiquement acceptable de celui-ci.
